# EUROPÄISCHE PATENTANMELDUNG

(11) **EP 0 793 956 A1**
(43) Veröffentlichungstag der Anmeldung: **10.09.1997**
(21) Anmeldenummer: 97102929.3
(22) Anmeldetag: 22.02.1997
(51) Int. Cl.: A61K 7/06

(54) **Haarkosmetische Zubereitungen auf der Grundlage von Phytosterolen und alpha-Hydroxycarbonsäuren**

(30) Priorität: 07.03.1996 DE 19608775
(71) Anmelder: Beiersdorf Aktiengesellschaft, D-20245 Hamburg (DE)
(72) Erfinder: Riedel, Jan-Henric, Dr., 22307 Hamburg (DE); Körbächer, Klaus, Dr., 20253 Hamburg (DE); Schmidt-Lewerkühne, Hartmut, Dr., 22869 Schenefeld (DE); Hengel, Roland, D-20459 Hamburg (DE)

(57) **Zusammenfassung**

Haarkosmetische Wirkstoffkombinationen aus
(a) einem oder mehreren Phytosterolen und
(b) einer oder mehreren α-Hydroxycarbonsäuren, und/oder α-Ketocarbonsäuren und/oder Salicylsäure

## Beschreibung

Die vorliegende Erfindung betrifft haarkosmetische Wirkstoffkombinationen und Zubereitungen, solche Kombinationen enthaltend. Insbesondere betrifft die vorliegende Erfindung haarkosmetische Wirkstoffkombinationen und Zubereitungen zur Pflege des Haars und der Kopfhaut. In einer bevorzugten Ausführungsform betrifft die vorliegende Erfindung Wirkstoffkombinationen und Zubereitungen, die dazu dienen, das einzelne Haar zu kräftigen und/oder der Haartracht insgesamt Halt und Fülle zu verleihen.

Das menschliche Haar kann, grob verallgemeinert, unterteilt werden in den lebenden Teil, die Haarwurzel, und den toten Teil, den Haarschaft. Der Haarschaft seinerseits besteht aus der Medulla, welche allerdings entwicklungsgeschichtlich bedingt für den neuzeitlichen Menschen unbedeutend geworden und zurückgebildet ist und bei dünnem Haar oft gänzlich fehlt, ferner dem die Medulla umschließenden Cortex und der die Gesamtheit aus Medulla und Cortex umhüllenden Cuticula.

Insbesondere die Cuticula, aber auch der keratinöse Bereich zwischen Cuticula und Cortex als Außenhülle des Haares sind besonderer Beanspruchung durch Umwelteinflüsse, durch Kämmen und Bürsten, aber auch durch Haarbehandlung, insbesondere Haarfärbung und Haarverformung, z.B. Dauerwellverfahren, ausgesetzt.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar. mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Hamsäure. Xanthin, Keratin, Glycogen, Citronensäure, Milchsäure) durch die Haarwäsche herausgelaugt werden.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Durch quaternäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Der Stande der Technik ließ es aber an wirkstoffen und Zubereitungen mangeln, welche dem geschädigten Haar in befriedigender Weise Pflege zukommen ließen. Auch erwiesen sich Zubereitungen, die der Haartracht Fülle geben sollten, oft als unzureichend, zumindest waren sie ungeeignet, als Haarpflegezubereitungen eingesetzt zu werden. Die Haartracht fixierende Zubereitungen des Standes der Technik enthalten beispielsweise in der Regel viskose Bestandteile, welche Gefahr laufen, ein Gefühl der Klebrigkeit zu erwecken, welches oft durch geschickte Formulierung kompensiert werden muß.

Aufgabe war daher, den Nachteilen des Standes der Technik Abhilfe zu schaffen.

Erstaunlicherweise hat sich herausgestellt, daß haarkosmetische Wirkstoffkombinationen aus
(a) einem oder mehreren Phytosterolen und
(b) einer oder mehreren α-Hydroxycarbonsäuren, und/oder α-Ketocarbonsäuren und/oder Salicylsäure
die Nachteile des Standes der Technik beseitigen.

Die erfindungsgemäßen Wirkstoffkombinationen und Zubereitungen, diese Wirkstoffkombinationen enthaltend, pflegen durch Umwelteinflüsse geschädigtes oder strapaziertes Haar bzw. beugen solchen Umwelteinflüssen vor. Zudem verbessern die erfindungsgemäßen Wirkstoffkombinationen und Zubereitungen, diese Wirkstoffkombinationen enthaltend, die Kämmbarkelt des Haares, ohne daß ein Zusatz an Wirkstoffen, enthaltend quaternäre Stickstoffgruppen ( 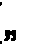 Quats 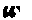 ) nötig wäre.

Phytosterole, auch Phytosterine genannt, sind Sterole, die in Pflanzen und Hefen gefunden werden. Letztere werden auch als Mykosterine bezeichnet. Im Gegensatze zu tierischen Sterinen sind Phytosterole in C-24-Position mit einem C₁ - oder C₂ - Rest substituiert und besitzen in C-22-Position meist eine Doppelbindung.

Beispiele für Phytosterole sind:

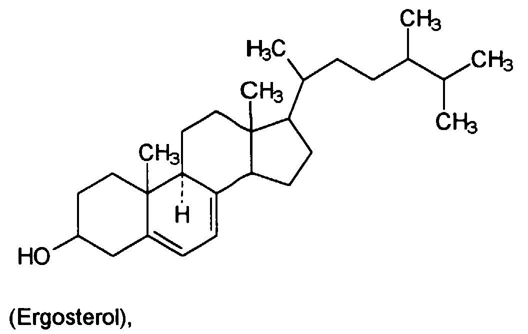

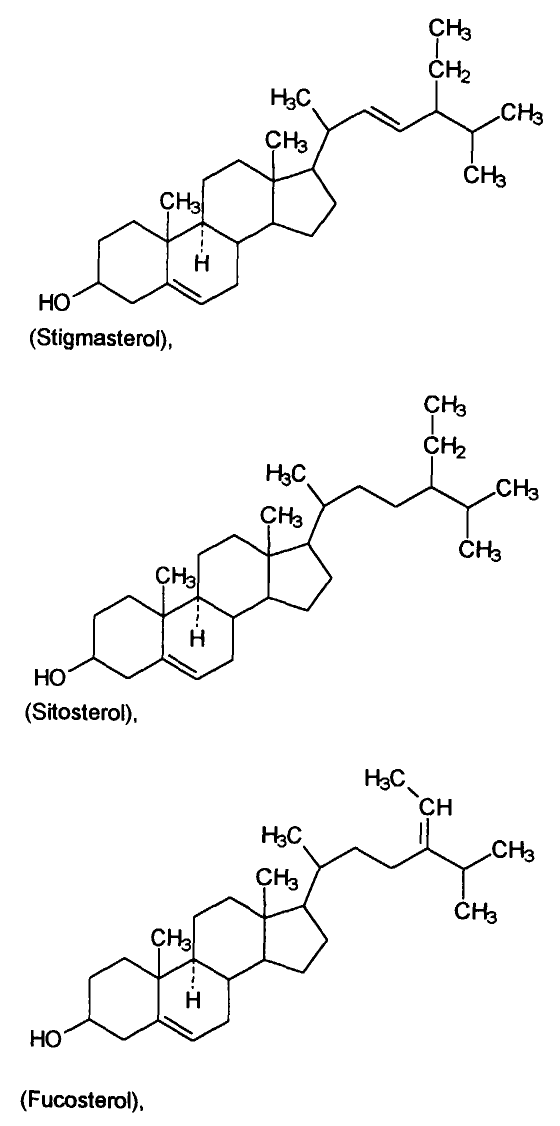

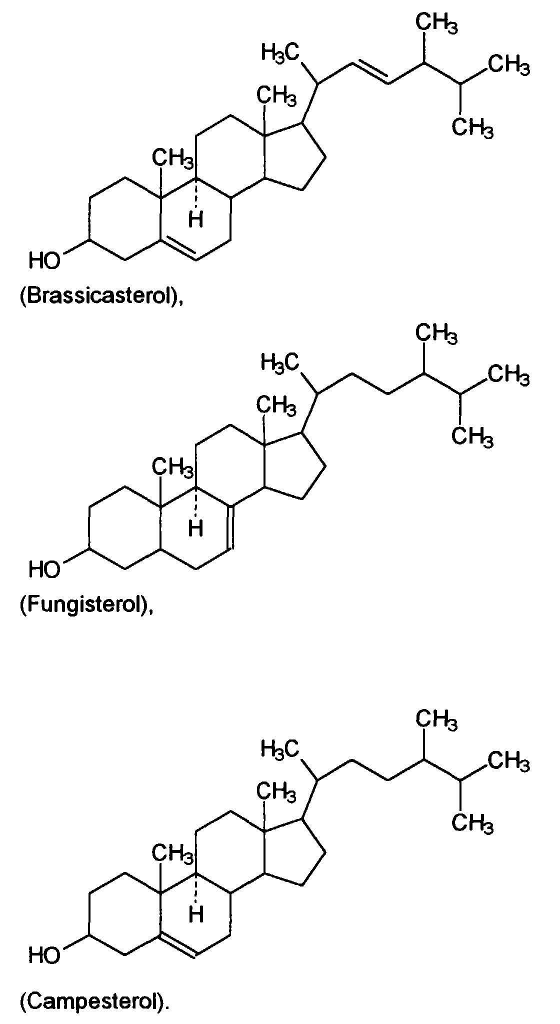

Alle erfindungsgemäß zu verwendenden Phytosterole besitzen eine mehr oder weniger große Anzahl an optischen Isomeren, welche hier im einzelnen nicht aufgeführt werden sollen, welche sich aber als vorteilhaft erwiesen haben, sofern ihre kosmetische Unbedenklichkeit außer Frage steht.

Bevorzugte Phytosterole sind Sitosterol, Campesterol, Stigmasterol.

Vorteilhaft enthalten erfindungsgemäße Zubereitungen 0,01 - 25 Gew.-% eines oder mehrerer Phytosterole, bevorzugt 0,1 - 20 Gew.-%, insbesondere 1 - 10 Gew.-%, jeweils bezogen auf das Gesamtgewicht der Zubereitungen.

Erfindungsgemäß wird oder werden die α-Hydroxycarbonsäure oder -säuren vorteilhaft gewählt aus der Gruppe der Substanzen der allgemeinen Formel 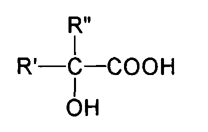 und/oder die α-Ketocarbonsäure oder -säuren wird oder werden gewählt aus der Gruppe der Substanzen der allgemeinen Formel 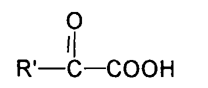 wobei jeweils R' und R'' unabhängig voneinander gewählt werden aus der Gruppe
(a1) H-,
(a2) verzweigtes oder unverzweigtes C₁₋₂₅₋Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes C₁₋₂₅-Alkyl-
(a4) Phenyl-,
(a5) mit einer oder mehreren Carboxylgruppen und/oder Hydroxygruppen und/oder verzweigten und/oder unverzweigten C₁₋₂₅-Alkylgruppen substituiertes Phenyl-,
   oder wobei das α-Kohlenstoffatom der α-Hydroxycarbonsäure mit R' und R'' zusammen eine
(a6) unsubstituierte Cycloalkylgruppe mit 3 bis 7 Ringatomen oder eine
(a7) mit einer oder mehreren Carboxylgruppen und/oder Hydroxygruppen und/oder Oxogruppen (Ketogruppen) und/oder verzweigten und/oder unverzweigten C₁₋₂₅-Alkylgruppen substituierte Cycloalkylgruppe mit 3 bis 7 Ringatomen ausbildet und wobei die α-Hydroxycarbonsäure oder die α-Hydroxycarbonsäuren oder die α-Ketocarbonsäure oder die α-Ketocarbonsäuren gegebenenfalls in Form ihrer physiologisch verträglichen Salze und/oder Ethylester und/oder Methylester vorliegen können.

Die Salicylsäure (auch 2-Hydroxybenzoäsäure, Spirsäure) ist durch die Struktur 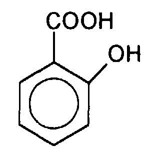 gekennzeichnet. Bekanntermaßen wirkt Salicylsäure antibakteriell und keratolytisch und ist Bestandteil mancher kosmetischen oder pharmazeutischen Zubereitungen.

Die erfindungsgemäßen α-Hydroxycarbonsäuren werden vorteilhaft gewählt aus folgenden Substanzklassen:
(a2) α-Hydroxyfettsäuren, wobei diese wiederum besonders vorteilhaft aus der Gruppe der C₁₀₋₁₈-Alkylcarbonsäuren gewählt werden,
(a3) α-Hydroxyzuckersäuren, aliphatische α-Hydroxyfruchtsäuren,
(a4) unsubstituierte aromatische α-Hydroxycarbonsäuren (z.B. Mandelsäure) bzw.
(a5) substituierte aromatische α-Hydroxycarbonsäuren.

Die unter Punkt (a2) fallenden α-Hydroxyfettsäuren werden besonders vorteilhaft gewählt aus der Gruppe
- α-Hydroxycarbonsäuren, gemäß der Formel 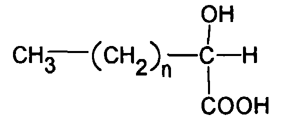 und/oder
- α-Hydroxy-isocarbonsäuren, gemäß der Formel 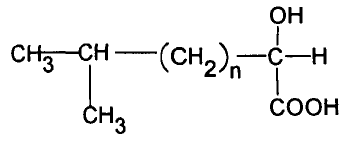 und/oder
- α-Hydroxy-anteisocarbonsäuren, gemäß der Formel 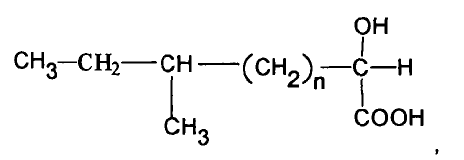 wobei n jeweils eine Zahl von 7 bis 31 darstellt.

Besonders vorteilhaft im Sinne der vorliegenden Erfindung ist, α-Hydroxycarbonsäuren zu verwenden, welche C₁₆-Körper darstellen, die also am α-Kohlenstoffatom eine verzweigte oder unverzweigte C₁₄H₂₉-Kette tragen.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (a3) fallenden α-Hydroxyzuckersäuren werden besonders vorteilhaft gewählt aus der Gruppe der
- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure.

Die unter Punkt (a3) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybernsteinsäure) ist durch folgende chemische Struktur gekennzeichnet:

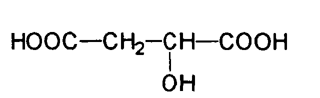

Milchsäure (2-Hydroxypropansäure) ist durch folgende chemische Struktur gekennzeichnet:

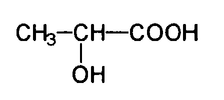

Citronensäure (2-Hydroxy-1,2,3-propantricarbonsäure) ist durch folgende chemische Struktur gekennzeichnet:

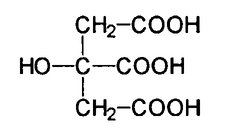

Bekanntermaßen wird Citronensäure zur Pufferung kosmetischer und/oder dermatologischer Zubereitungen, aber auch als Synergist für Antioxidantien in der Haut- und Haarkosmetik verwendet.

Weinsäure (Dihydroxybernsteinsäure) ist durch folgende chemische Struktur gekennzeichnet:

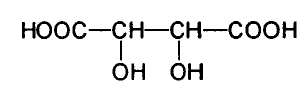

Bevorzugte α-Ketocarbonsäure ist die Brenztraubensäure (α-Oxopropansäure). Sie zeichnet sich durch folgende Struktur aus:

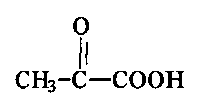

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Vorteilhaft enthalten erfindungsgemäße Zubereitungen neben einem wirksamen Gehalt an erfindungsgemäßen Wirkstoffkombinationen ferner übliche Wirk-, Inhalts-, Zusatz- und/oder Hilfsstoffe.

Besonders vorteilhaft ist, den erfindungsgemäßen Zubereitungen Chitosan zuzusetzen.

Chitosan ist gekennzeichnet durch folgende Strukturformel: 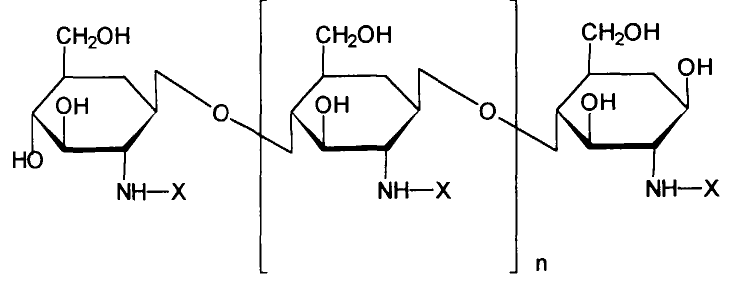 dabei nimmt n Werte bis zu ca. 2.000 an. X stellt entweder den Acetylrest oder Wasserstoff dar. Chitosan entsteht durch Deacetylierung und teilweise Depolymerisation (Hydrolyse) von Chitin, welches durch die Strukturformel 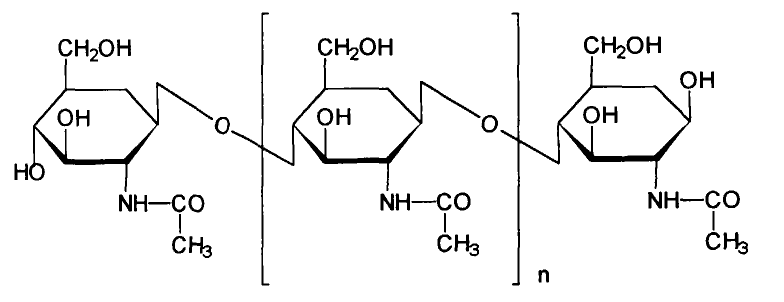 gekennzeichnet ist. Chitin ist wesentlicher Bestandteil des Ektoskeletts [ 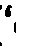 ο χ 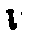 των = grch.: der Panzerrock] der Gliederfüßer (z.B. Insekten, Krebse, Spinnen) und wird auch in Stützgeweben anderer Organismen (z.B. Weichtiere, Algen. Pilze) gefunden.

Chitosan ist ein in der Haarpflege bekannter Rohstoff. Es eignet sich, besser als das ihm zugrundeliegende Chitin. als Verdicker oder Stabilisator und verbessert die Adhäsion und Wasserresistenz von polymeren Filmen. Stellvertretend für eine Vielzahl von Fundstellen des Standes der Technik: H.P.Fiedler. 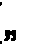 Lexikon der Hilfsstoffe für Pharmazie, Kosmetik und angrenzende Gebiete 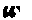 , dritte Auflage 1989, Editio Cantor, Aulendorf, S. 293, Stichwort 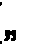 Chitosan 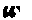 .

Erfindungsgemäß bevorzugt sind Chitosane mit einem Deacetylierungsgrad > 60 %, insbesondere > 80%. Unter diesen besonders bevorzugt sind solche, deren 1%-ige wäßrige Lösung eine Viskosität von 4.500 - 5.500 mPas (Brookfield, Spindel 5, 10 UpM), insbesondere 5.000 mPas aufweist.

Vorteilhaft enthalten die erfindungsgemäßen Zubereitungen 0,05 - 5 Gew.-% Chitosan, bevorzugt 0,5 - 2 Gew.-%, insbesondere 1 Gew.-%, jeweils bezogen auf das Gesamtgewicht der Zubereitungen.

Obwohl es erfindungsgemäß möglich ist, auf quaternäre Stickstoffverbindungen ( 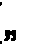 Quats 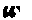 ) zu verzichten, kann sich ein Zusatz davon gegebenenfalls als vorteilhaft erweisen.

Als filmbildendes Polymer mit wenigstens teilweise quaternisierten Stickstoffgruppen (im folgenden 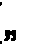 Filmbildner 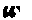 genannt, eigenen sich bevorzugt solche, welche gewählt werden aus der Gruppe der Substanzen, welche nach der INCI-Nomenklatur (International Nomenclature Cosmetic Ingredient) den Namen 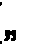 Polyquaternium 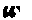 tragen, beispielsweise:
- Polyquaternium-2: (Chemical Abstracts-Nr. 63451-27-4, z.B. Mirapol® A-15)
- Polyquaternium-5: (Copolymeres aus dem Acrylamid und dem β-Methacryloxyethyltrimethylammoniummethosulfat, CAS-Nr. 26006-22-4)
- Polyquaternium-6: (Homopolymer des N,N-Dimethyl-N-2-propenyl-2-propen-1-aminiumchlorids, CAS-Nr. 26062-79-3, z.B. Merquat® 100
- Polyquaternium-7: N,N-Dimethyl-N-2-propenyl-2-propen-1-aminiumchlorid, Polymeres mit 2-Propenamid, CAS-Nr. 26590-05-6, z.B. Merquat® S
- Polyquaternium-10: Quaternäres Ammoniumsalz der Hydroxyethylcellulose, CAS-Nr. 53568-66-4, 55353-19-0, 54351-50-7, 68610-92-4, 81859-24-7, z.B. Celquat® SC-230M,
- Polyquaternium-11: Vinylpyrrolidon/dimethylaminoethyl-Methacrylat-Copolymer/Diethylsulfat-Reaktionsprodukt, CAS-Nr. 53633-54-8, z.B. Gafquat® 755N
- Polyquaternium-16: Vinylpyrrolidon/vinylimidazoliniummethochlorid-Copolymer, CAS-Nr. 29297-55-0, z.B. Luviquat® HM 552
- Polyquaternium-17: CAS-Nr. 90624-75-2, z.B. Mirapol® AD-1
- Polyquaternium-19: Quaternisierter wasserlöslicher Polyvinylalkohol
- Polyquaternium-20: in Wasser dispergierbarer quaternisierter Polyvinyloctadecylether
- Polyquaternium-21: Polysiloxan-polydimethyl-dimethylammoniumacetat-Copolymeres, z.B. Abil® B 9905
- Polyquaternium-22: Dimethyldiallylammoniumchlorid/Acrylsäure-Copolymer, CAS-Nr. 53694-7-0, z.B. Merquat® 280
- Polyquaternium-24: Polymeres quaternäres Ammoniumsalz der Hydroxyethylcellulose, Reaktionsprodukt mit einem mit Lauryldimethylammonium substituierten Epoxid, CAS-Nr. 107987-23-5. z.B. Quatrisoft® LM-200
- Polyquaternium-28: Vinylpyrrolidon/Methacrylamidopropyltrimethylammoniumchlorid-Copolymer, z.B. Gafquat® HS-100
- Polyquaternium-29: z.B. Lexquat® CH
- Polyquaternium-31: CAS-Nr. 136505-02-7, z.B. Hypan® QT 100
- Polyquaternium-32: N,N,N-trimethyl-2-[(2-methyl-1-oxo-2-propenyl)oxy]-Ethanaminiumchlorid, polymer mit 2-Propenamid, CAS-Nr. 35429-19-7
- Polyquaternium-37: CAS-Nr. 26161-33-1

Erfindungsgemäß bevorzugter Filmbildner ist Polyquaternium-11.

Vorteilhaft enthalten erfindungsgemäße Zubereitungen 0.2 - 50 Gew.-% eines oder mehrerer Filmbildner, bevorzugt 5 - 30 Gew.-%, insbesondere 10 - 25 Gew.-%, jeweils bezogen auf das Gesamtgewicht der Zubereitungen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Insbesondere können die erfindungsgemäßen kosmetischen und dermatologischen Zubereitungen vorteilhaft Antioxidantien enthalten.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die wäßrige erfindungsgemäßen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0.01 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UVB-Filtersubstanzen, können diese vorteilhaft wasserlöslich sein. Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Es kann auch von Vorteil sein,erfindungsgemäße Zubereitungen mit UVA-Filtern zu versetzen, die bisher üblicherweise in kosmetischen Zubereitungen enthalten sind. Es können die für die UVB-Kombination verwendeten Mengen eingesetzt werden.

Bei kosmetischen und dermatologischen Zubereitungen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Shampoonierungsmittel darstellen, enthalten vorzugsweise mindestens eine anionische, nicht-ionische oder amphotere oberflächenaktive Substanz, oder auch Gemische aus solchen Substanzen, Chlorogensäure im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrigalkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält die erfindungsgemäßen Kombinationen.

Erfindungsgemäß können kosmetische Zubereitungen zur Behandlung und Pflege der Haare als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Kosmetische Zubereitungen, die kosmetische Reinigungszubereitungen für das Haar bzw. die Kopfhaut darstellen, können in flüssiger oder fester Form vorliegen. Sie enthalten vorzugsweise mindestens eine anionische, nicht-ionische oder amphotere oberflächenaktive Substanz oder Gemische daraus, gegebenenfalls einen Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Die erfindungsgemäßen Zusammensetzungen enthalten gegebenenfalls die in der Kosmetik üblichen Zusatzstoffe, beispielsweise Parfüm. Verdicker. Farbstoffe, Desodorantien, antimikrobielle Stoffe, rückfettende Agentien. Komplexierungs- und Sequestrierungsagentien, Perlglanzagentien, Pflanzenextrakte, Vitamine, Wirkstoffe und dergleichen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

| 1) Haarlotion | | | | |
|---|---|---|---|---|
| | I | II | III | IV |
| Alkohol | 50 | 50 | 50 | 50 |
| Milchsäure | 0,50 | 1,0 | 1,5 | 2,0 |
| Campesterol | 0,50 | - | - | - |
| Ergosterol | - | 0,50 | - | - |
| Sitosterol | - | - | 0,50 | - |
| Stigmasterol | - | - | - | 0,50 |
| Konservierungsmittel, Parfüm, Farbstoffe | q.s. | q.s. | q.s. | q.s. |
| Wasser, VES | ad 100,00 | ad 100,00 | ad 100,00 | ad 100,00 |

| 2) Haarkur | | |
|---|---|---|
| | I | II |
| Hydroxypropylmethylcellulose | 0,50 | 0,50 |
| Glycerin | 6,50 | 6,50 |
| Cetearylalkohol | 3,50 | 3,50 |
| Glycerylstearat | 3,00 | 3,00 |
| Milchsäure | 2,00 | 3,00 |
| Ergosterol | 0,50 | - |
| Stigmasterol | | 1,00 |
| Konservierungsmittel, Parfum, Farbstoffe | q.s. | q.s. |
| Wasser, VES | ad 100,00 | ad 100,00 |

| 3) Haarspülung | | |
|---|---|---|
| | I | II |
| Glycerin | 5,00 | 5,00 |
| Hydroxyethylcellulose | 0,20 | 0,20 |
| Cetearylalkohol | 5,00 | 5,00 |
| Milchsäure | 1,50 | 2,00 |
| Campesterol | 0,50 | - |
| Stigmasterol | | 0,90 |
| Konservierungsmittel, Parfum, Farbstoffe | q.s. | q.s. |
| Wasser, VES | ad 100,00 | AD 100,00 |

| 4) Shampoo | | |
|---|---|---|
| | I | II |
| Natriumlaurethsulfat | 12,00 | 12,00 |
| Cocoamidopropylbetain | 3,00 | 3,00 |
| Dinatriumlaurethsulfosuccinat | 1,50 | 1,50 |
| Milchsäure | 2,00 | 3,00 |
| Sitosterol | 1,00 | - |
| Campesterol | - | 1,50 |
| Perlglanzmittel | 4,00 | 4,00 |
| Konservierungsmittel, Parfum, Farbstoffe, Verdicker | q.s. | q.s. |
| Wasser, VES | ad 100,00 | ad 100,00 |

## Patentansprüche

1. Haarkosmetische Wirkstoffkombinationen aus
(a) einem oder mehreren Phytosterolen und
(b) einer oder mehreren α-Hydroxycarbonsäuren, und/oder α-Ketocarbonsäuren und/oder Salicylsäure.

2. Zubereitungen nach Anspruch 1, dadurch gekennzeichnet, daß das oder die Phytosterole gewählt wird oder werden aus der Gruppe der Sitosterole, Campesterole, Stigmasterole.

3. Zubereitungen nach Anspruch 1, enthaltend 0,05 - 5 Gew.-% Phytosterole, bevorzugt 0.5 - 2 Gew.-%, insbesondere 1 Gew.-%, jeweils bezogen auf das Gesamtgewicht der Zubereitungen.

4. Zubereitungen nach Anspruch 1, enthaltend 0,01 - 25 Gew.-% einer oder mehrerer α-Hydroxycarbonsäuren, bzw. α-Ketocarbonsäuren bzw. Salicylsäure, bevorzugt 0,1 - 20 Gew.-%, insbesondere 1 - 10 Gew.-%, jeweils bezogen auf das Gesamtgewicht der Zubereitungen.
